(19) Europäisches Patentamt
European Patent Office
Office européen des brevets

(11) EP 4 262 745 B1

(12) EUROPEAN PATENT SPECIFICATION

(45) Date of publication and mention of the grant of the patent:
**29.01.2025 Bulletin 2025/05**

(21) Application number: **21836185.5**

(22) Date of filing: **16.12.2021**

(51) International Patent Classification (IPC):
***A61K 38/26** (2006.01)* ***A61K 9/00** (2006.01)*
***A61K 47/10** (2017.01)* ***A61K 47/18** (2017.01)*
***A61K 47/26** (2006.01)*

(52) Cooperative Patent Classification (CPC):
**A61K 9/0019; A61K 38/26; A61K 47/10; A61K 47/18; A61K 47/26**

(86) International application number:
**PCT/EP2021/086147**

(87) International publication number:
**WO 2022/129311 (23.06.2022 Gazette 2022/25)**

# (54) PHARMACEUTICAL COMPOSITION OF GLP-1/GLP-2 DUAL AGONISTS

PHARMAZEUTISCHE ZUSAMMENSETZUNG VON DUALEN GLP-1-/GLP-2-AGONISTEN

COMPOSITION PHARMACEUTIQUE D'AGONISTES DOUBLES DE GLP-1/GLP-2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2020 EP 20214562**

(43) Date of publication of application:
**25.10.2023 Bulletin 2023/43**

(73) Proprietor: **Zealand Pharma A/S**
**2860 Søborg (DK)**

(72) Inventors:
- **VILLADSEN, Jesper Skodborg**
  **2860 Søborg (DK)**
- **GIEHM, Lise**
  **2860 Søborg (DK)**

(74) Representative: **D Young & Co LLP**
**3 Noble Street**
**London EC2V 7BQ (GB)**

(56) References cited:
**WO-A1-2005/046716 WO-A1-2018/104561**
**WO-A1-2020/249778**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present invention relates to pharmaceutical compositions comprising particular preservatives. The pharmaceutical compositions according to the invention are particularly stable, and have an advantageous shelf-life.

### BACKGROUND OF THE INVENTION

Peptides are an important segment of the pharmaceutical industry. Although there have been tremendous advances in production of the active pharmaceutical ingredient (API), production of peptide-based drug products is still a significant challenge. Challenges in connection with peptide formulation development are often over-looked or neglected.

In general, peptides are defined as polypeptides of less than 50 amino residues and are often lacking organised tertiary or globular structure. Some do adopt secondary structures, although this tends to be limited, for example a single turn of an $\alpha$-helix. While their smaller size makes them easier to deliver across biological barriers than larger proteins, their formulation can be problematic.

Some of the formulation challenges relating to peptides in particular include: chemical instability; adopting multiple conformers; their tendency to self-associate; and a complex physical instability, such as gel formation, amyloid formation and/or precipitation.

The most common challenge is chemical degradation of peptides and proteins, through degradation mechanisms such as isomerization, racemization, hydrolysis, deamidation and oxidation. The amino acid sequence of a given peptide defines to what extent it is affected by deamidation and/or oxidation reactions. Oxidation rates of specific residues, such as Met residues, correlate with the degree of solvent exposure. As peptides do not possess a globular structure that can sequester reactive groups, the side chains of nearly all of the residues in a peptide are fully solvent exposed, allowing maximal contact with reactive oxygen species. Deamidation involves hydrolysis of the amide sidechain of amino acid residues, such as Asn and Gln. Further, the high degree of peptide chain flexibility leads to high rates of deamidation, compared to more complex proteins. It is however important to note that the nature of the amino acid following the deamidation, e.g. the one following Asn, also impacts deamidation rates. A peptides lack of steric bulk and the ability to hydrogen bond to the Asn side chain may even speed up the reaction further. Typically, Asn-Gly, Asn-Ala, Asn-Ser and Asn-Asp amino acid combinations display reaction rates that scientists have to factor in and test to ensure stable pharmaceutical compositions. The greatest control over hydrolytic reactions, including deamidation, is exerted by stable and reliable pH and buffer systems. Such stable and reliable pH and buffer systems will however be affected by additional excipients added to the composition.

Excipients are added to pharmaceutical compositions to enhance or maintain active ingredient solubility (solubilisers) and/or stability (buffers, antioxidants, chelating agents, cryo- and lyoprotectants). Excipients are in many instances important in parenteral formulations to assure safety (antimicrobial preservatives), minimise pain and irritation upon injection (tonicity agents), and control or prolong drug delivery (polymers). These are all examples of positive or synergistic interactions between excipients and medicinal products. However, any excipient added to the composition has the potential to produce negative effects such as loss of peptide solubility, activity, and/or chemical/physical stability, increased self-aggregation or fibrillation, which in turn may render the medicinal product unsafe for administration.

Preservatives may be added to pharmaceutical compositions to kill microorganism contaminants that may be introduced into the composition, such as when multiple aliquots are used or withdrawn from a container holding multiple doses of a medicament. Pharmaceutical compositions can be sealed and stored in sterile conditions without preservatives being present, but when the container holding the composition is used, any accidental introduction of microorganisms can render the contents unsuitable for further medical use. Therefore, it is important to effectively preserve the pharmaceutical contents, especially when the composition is stored in a large volume for several administrations. If a container holding a large volume of an unpreserved pharmaceutical composition is used, the lack of a preservative may mean the majority of the contents are wasted. Preservatives advantageously enable storage of pharmaceutical compositions, such as for many months or years, at low temperatures (e.g. refrigerated at around 5 °C), or storage for shorter periods, such as a few days or weeks, at higher temperatures, such as room temperature, even after part of the composition has been used.

However, despite the advantages of using preservatives, the inclusion of a preservative in a pharmaceutical composition may be problematic as the preservative may interact detrimentally with other components of the composition, in particular the active component. Such interactions can lead to a reduced preservative effect, or a reduced or complete lack of medical efficacy of the pharmaceutical composition. For example, the preservative may cause chemical instability of the active. In the case of a peptide active, a preservative may participate in or promote degradative reactions such as isomerization, racemization, hydrolysis, deamidation or oxidation of the peptide, resulting in loss of pharmacological activity of the peptide. Alternatively or additionally, a preservative may be detrimental to the physical stability of a peptide active, enhancing aggregation of the peptide into inactive covalent oligomers and/or causing the peptide to precipitate out

of solution. Not only does such loss of physical stability reduce the medical potency of the peptide, but also formation of particulate matter has practical and safety implications if the composition is delivered by injection.

Given the great sequence variety, and thus different chemical structures, of peptides, it is inherently unpredictable whether a given substance will act as an effective preservative for a particular therapeutic peptide composition without negatively affecting the peptide in the ways described above.

The present invention concerns pharmaceutical compositions comprising selected peptides disclosed in WO2018104561 (e.g. compound 18 of WO2018104561), which describes the compounds and their uses in detail.

## SUMMARY OF THE INVENTION

This application provides compositions comprising one or more GLP-1/GLP-2 dual agonist and one or more preservative in a buffer, as defined by the claims.

In some aspects, the compositions are isotonic parenteral pharmaceutical compositions suitable for administration to human subjects.

The GLP-1/GLP-2 dual agonist is a peptide. Particular preservatives have been identified that can be used in a composition comprising a specific GLP-1/GLP-2 dual agonist peptide and a particular buffer without substantially impacting the chemical or physical stability of the peptide. It has been surprisingly found that particular preservatives have little to no effect on chemical stability and oligomerisation of the peptide in tris buffer.

The compositions of the invention therefore benefit from the advantages associated with preservatives, namely that contamination with microorganisms is prevented or reduced.

Of particular advantage, the preservative effect in the composition of the invention allows the composition to be provided in a multi-dose administration setup. The composition of the invention can be provided in a device that is used to administer sequential therapeutic doses of the composition at intervals over an extended period. The preservative effect prevents growth of microorganisms in the composition over this period, whilst chemical and physical stability of the peptide is maintained. This has the practical benefit of needing to load the device only once, instead of preparing a new dose for each administration.

In one aspect the pharmaceutical compositions according to the invention are for, or suitable for, administration in a multi-dose device.

In some aspects, the invention provides a composition comprising:

(a) one or more GLP-1/GLP-2 dual agonist comprising general formula A:

H[Aib]EG-X5-F-X7-SELATILD-[Ψ]-QAARDFIAWLI-X28-HKITD (A),

wherein X5 is T or S; X7 is T or S; X28 is Q, E, A, H, Y, L, K, R or S and at least one of X5 and X7 is T,

wherein [Ψ] indicates an L or D lysine residue in which an albumin binding moiety is conjugated to the GLP-1/GLP-2 dual agonist, and

wherein said albumin binding moiety is [K([17-carboxy-heptadecanoyl]-isoGlu)];

(b) one or more preservative,
wherein the one or more preservative comprises or is m-cresol and/or phenol; and
(c) tris buffer.

In some aspects, particular and specific compositions, such as isotonic parenteral compositions, are described in detail in the description of the invention.

The chemical stability at time point Y of GLP-1/GLP-2 dual agonist in any of the tested compositions disclosed herein can be expressed as the relative purity $X^Y$ of the GLP-1/GLP-2 dual agonist and is determined by measuring the absolute purity X' of the GLP-1/GLP-2 dual agonist and normalising it to the absolute purity X° of the GLP-1/GLP-2 dual agonist at day zero (day 0), wherein said absolute purities are determined by HPLC at a given time point Y by identifying the purity of peak corresponding to the GLP-1/GLP-2 dual agonist.

It was surprisingly found that the peptide of the invention is more stable and oligomerises less when m-cresol preservative is combined with tris buffer rather than phosphate buffer.

The invention also provides the composition according to the invention for use in:

(i) increasing intestinal mass, improving intestinal function, increasing intestinal blood flow, or repairing intestinal

damage or dysfunction, in a subject in need thereof; or

(ii) the prophylaxis or treatment of malabsorption, ulcers, short-bowel syndrome, cul-de-sac syndrome, inflammatory bowel disease, irritable bowel syndrome, pouchitis, celiac sprue, tropical sprue, hypogammaglobulinemic sprue, mucositis induced by chemotherapy or radiation therapy, diarrhoea induced by chemotherapy or radiation therapy, low grade inflammation, metabolic endotoxemia, necrotising enterocolitis, primary biliary cirrhosis, hepatitis, fatty liver disease, or gastrointestinal side-effects of inflammatory conditions, in a subject in need thereof; or

(iii) reducing or inhibiting weight gain, reducing gastric emptying or intestinal transit, reducing food intake, reducing appetite, or promoting weight loss, in a subject in need thereof; or

(iv) the prophylaxis or treatment of obesity, morbid obesity, obesity-linked gallbladder disease, obesity-induced sleep apnoea, inadequate glucose control, glucose tolerance, dyslipidaemia, diabetes, pre-diabetes, metabolic syndrome or hypertension, in a subject in need thereof.

The invention further provides a method for preserving a composition comprising the one or more GLP-1/GLP-2 dual agonist of the invention and tris buffer, wherein the method comprises adding one or more preservative to the composition, wherein the wherein the one or more preservative comprises or is m-cresol and/or phenol.

The invention additionally provides use of a preservative for preserving a composition comprising the one or more GLP-1/GLP-2 dual agonist of the invention and tris buffer, wherein the preservative comprises or is m-cresol and/or phenol.

**DESCRIPTION OF THE INVENTION**

**Compounds**

The composition of this invention comprises one or more GLP-1/GLP-2 dual agonist comprising general formula A:

H[Aib]EG-X5-F-X7-SELATILD-[Ψ]-QAARDFIAWLI-X28-HKITD (A),

wherein X5 is T or S; X7 is T or S; X28 is Q, E, A, H, Y, L, K, R or S and at least one of X5 and X7 is T, wherein [Ψ] indicates an L or D lysine residue in which an albumin binding moiety is conjugated to the GLP-1/GLP-2 dual agonist, and wherein said albumin binding moiety is [K([17-carboxy-heptadecanoyl]-isoGlu)].

In some aspects, the one or more GLP-1/GLP-2 dual agonist comprising general formula A is of the general formula B:

H[Aib]EG-X5-FT-SELATILD-[Ψ]-QAARDFIAWLI-X28-HKITD (B),

wherein X5 is T or S and X28 is Q, E, A, H, Y, L, K, R or S, wherein [Ψ] indicates an L or D lysine residue in which the albumin binding moiety is conjugated to the GLP-1/GLP-2 dual agonist, and wherein said albumin binding moiety is [K([17-carboxy-heptadecanoyl]-isoGlu)].

In some aspects, the one or more GLP-1/GLP-2 dual agonist comprising general formula A or B comprises the sequence: H[Aib]EGSFTSELATILD[Ψ]QAARDFIAWLIQHKITD (SEQ ID NO: 1). In some aspects, the one or more GLP-1/GLP-2 dual agonist comprising general formula A or B consists of the sequence: H[Aib]EGSFTSELATILD[Ψ]QAARDFIAWLIQHKITD (SEQ ID NO: 1).

In some aspects, the one or more GLP-1/GLP-2 dual agonist comprising general formula A is: Hy-H[Aib]EGSFTSELATILD[K([17-carboxy-heptadecanoyl]-isoGlu)]QAARDFIAWLIQHKITD-OH (CPD1OH), or any pharmaceutical acceptable salt thereof. In some aspects, the pharmaceutically acceptable salt of CPD1OH is a sodium salt, a chloride salt or an acetate salt, preferably a chloride salt.

In some aspects, the one or more GLP-1/GLP-2 dual agonist comprising general formula A is: Hy-H[Aib]EGSFTSELATILD[K([17-carboxy-heptadecanoyl]-isoGlu)]QAARDFIAWLIQHKITD-$NH_2$ (CPD1$NH_2$), or any pharmaceutical acceptable salt thereof. In some aspects, the pharmaceutically acceptable salt of CPD1$NH_2$ is a sodium salt, a chloride salt or an acetate salt, preferably a chloride salt.

In a preferred aspect the one or more GLP-1/GLP-2 dual agonist is CPD1OH or any pharmaceutical acceptable salt thereof, preferably a chloride salt thereof.

The term "GLP-1/GLP-2 dual agonist" as used herein refers to a peptide which has activity on the GLP-1 receptor and the GLP-2 receptor. A GLP-1/GLP-2 dual agonist comprising formula A or B may be a peptide of SEQ ID NO:1 or a

peptide wherein one or more amino acids have been modified relative to SEQ ID NO: 1. Such agonists and/or peptides may further comprise one or more side chains, which have been covalently attached to the GLP-1/GLP-2 dual agonist. The term "side chain" may also be referred to as a "substituent".

The term "salts" as used herein refers to an ionic compound that can be formed by the neutralisation reaction of an acid and a base. Salts are composed of related numbers of cations (positively charged ions) and anions (negative ions) so that the product is electrically neutral (without a net charge). These component ions can be inorganic, such as chloride ($Cl^-$), or organic, such as acetate ($CH_3CO^{2-}$) ; and can be monatomic, such as fluoride ($F^-$), or polyatomic, such as sulfate ($SO_4^{2-}$).

The terms "pharmaceutically acceptable salt of CPD1" or "salt of CPD1" as used herein describe salts of the compound comprising SEQ ID NO: 1. "Hy-H[Aib]EGSFTSELATILD[K([17-carboxy-heptadecanoyl]-isoGlu)]QAARDFIAWLIQHKITD-OH. [acid]" as used herein identifies a salt of Hy-H[Aib]EGSFTSELATILD[K([17-carboxy-heptadecanoyl]-isoGlu)]QAARDFIAWLIQHKITD-OH, wherein [acid] refers to the acid, which in a neutralisation reaction forms the salt of said compound, e.g. Hy-H[Aib]EGSFTSELATILD[K([17-carboxy-heptadecanoyl]-isoGlu)]QAARDFIAWLIQHKITD-OH.[HCl] will thus refer to a chloride salt.

"Pharmaceutically acceptable salt" as used herein refers to salts that are safe and effective for use in mammals and that possess the desired biological activity. Pharmaceutically acceptable salts include salts of acidic or basic groups present in CPD1. For a review on pharmaceutically acceptable salts, see Berge et al., 66 J. Pharm. Sci. 1-19 (1977).

***Table 1 - Selected GLP-1/GLP-2 dual agonist comprised in the composition of this invention***

| SEQ ID | CPD | CPD form | Compound |
|---|---|---|---|
| **1** | 1 | 1OH | Hy-H[Aib]EGSFTSELATILD[K([17-carboxy-heptadecanoyl]-isoGlu)]QAARDFIAWLIQHKITD-OH or any acceptable pharmaceutical salt thereof. |
| **1** | 1 | $1NH_2$ | Hy-H[Aib]EGSFTSELATILD[K([17-carboxy-heptadecanoyl]-isoGlu)]QAARD-FIAWLIQHKITD-$NH_2$ or any acceptable pharmaceutical salt thereof. |

The abbreviation CPD1 refers to any form of the compound comprising SEQ ID NO: 1. However CPD1OH solely discloses the compound comprising SEQ ID NO: 1, wherein said compound is in its -OH form (free acid). CPD1$NH_2$ form refers to the compounds -$NH_2$ form (amidated form). Both CPD1OH and CPD1$NH_2$ can be converted into a pharmaceutical acceptable salt to provide a drug substance in powder form.

***Table 2 - Amino acid sequence comprised in one or more GLP-1/GLP-2 dual agonist of the present invention***

| SEQ ID | Sequence | Variables |
|---|---|---|
| 1 | H[Aib]EGSFTSELATILD[Ψ]QAARDFIAWLIQHKITD | None |
| 2 (Formula A) | H[Aib]EG-X5-F-X7-SELATILD-[Ψ]-QAARDFIAWLI-X28-HKITD | X5 is T, S; X7 is TorS; X28 is Q, E, A, H, Y, L, K, R or S |
| 3 (Formula B) | H[Aib]EG-X5-FT-SELATILD-[Ψ]-QAARDFIAWLI-X28-HKITD | X5 is T, S; X28 is Q, E, A, H, Y, L, K, R or S |

The "albumin binding moiety" promotes the circulation of the GLP-1/GLP-2 dual agonist within the blood stream, and also has the effect of prolonging the time of action of the GLP-1/GLP-2 dual agonist. The albumin binding moiety binds the GLP-1/GLP-2 dual agonist to the albumin present in the blood and due to the fact that the GLP-1/GLP-2 dual agonist is only slowly released from albumin the action of the GLP-1/GLP-2 dual agonist is prolonged. The term "albumin binding moiety" may also be referred to as "side chain" or "substituent.

When used herein the term "natural amino acid" is an amino acid (with the usual three letter codes and one letter codes in parenthesis) selected from the group consisting of: Glycine (Gly & G), proline (Pro & P), alanine (Ala & A), valine (Val & V), leucine (Leu & L), isoleucine (Ile & !), methionine (Met & M), cysteine (Cys & C), phenylalanine (Phe & F), tyrosine (Tyr & Y), tryptophan (Trp & W), histidine (His & H), lysine (Lys & K), arginine (Arg & R), glutamine (Gln & Q), asparagine (Asn & N), glutamic acid (Glu & E), aspartic acid (Asp & D), serine (Ser & S) and threonine (Thr & T). If not otherwise indicated amino acids indicated with a single letter code in CAPITAL letters indicate the L-isoform, if however, the amino acid is indicated with a lower case letter, this amino acid is used/applied as it's D-form, e.g. K (i.e. L-lysine), k (i.e. D-lysine).

The abbreviation "Hy-" in connection with the compounds disclosed herein refers to hydrogen. The abbreviation in chosen to be indicated as "Hy" to avoid the hydrogen to be confused with the Histidine (H) in the beginning of the sequence.

Throughout the present description and claims, the generally accepted three-letter codes for other "α-amino acids" are used, such as sarcosine (Sar), norleucine (Nle), α-aminoisobutyric acid (Aib), 2,3-diaminopropanoic acid (Dap), 2,4-diaminobutanoic acid (Dab) and 2,5-diaminopentanoic acid (ornithine; Orn). Such other α-amino acids may be shown in square brackets "[ ]" (e.g. "[Aib]") when used in a general formula or sequence in the present specification, especially when the rest of the formula or sequence is shown using the single letter code.

***Concentration of compound***

In some aspects, a composition of this invention, such as an isotonic parenteral pharmaceutical composition of this invention, comprises at least about 1 mg/mL of the GLP-1/GLP-2 dual agonist, such as at least about 2 mg/mL of the GLP-1/GLP-2 dual agonist. In some aspects, a composition of this invention, such as an isotonic parenteral pharmaceutical composition of this invention, comprises from at least about 1 mg/mL to about 33 mg/mL of the GLP-1/GLP-2 dual agonist, such as from at least about 2 mg/mL to about 33 mg/mL of the GLP-1/GLP-2 dual agonist. In some aspects, a composition of this invention, such as an isotonic parenteral pharmaceutical composition of this invention, comprises from at least about 1 mg/mL to about 25 mg/mL of the GLP-1/GLP-2 dual agonist, such as from at least about 2 mg/mL to about 25 mg/mL of the GLP-1/GLP-2 dual agonist, such as from at least about 4 mg/mL to about 25 mg/mL of the GLP-1/GLP-2 dual agonist, such as from at least about 6 mg/mL to about 25 mg/mL of the GLP-1/GLP-2 dual agonist, such as from at least about 8 mg/mL to about 25 mg/mL of the GLP-1/GLP-2 dual agonist, such as from at least about 10 mg/mL to about 25 mg/mL of the GLP-1/GLP-2 dual agonist.

In some aspects, a composition of this invention, such as an isotonic parenteral pharmaceutical composition of this invention, comprises about 1 mg/mL GLP-1/GLP-2 dual agonist. In some aspects, a composition of this invention, such as an isotonic parenteral pharmaceutical composition of this invention, comprises about 2 mg/mL GLP-1/GLP-2 dual agonist. In some aspects, a composition of this invention, such as an isotonic parenteral pharmaceutical composition of this invention, comprises about 4 mg/mL GLP-1/GLP-2 dual agonist. In some aspects, a composition of this invention, such as an isotonic parenteral pharmaceutical composition of this invention, comprises about 6 mg/mL GLP-1/GLP-2 dual agonist. In some aspects, a composition of this invention, such as an isotonic parenteral pharmaceutical composition of this invention, comprises about 8 mg/mL GLP-1/GLP-2 dual agonist. In some aspects, a composition of this invention, such as an isotonic parenteral pharmaceutical composition of this invention, comprises about 10 mg/mL GLP-1/GLP-2 dual agonist. In some aspects, a composition of this invention, such as an isotonic parenteral pharmaceutical composition of this invention, comprises about 15 mg/mL GLP-1/GLP-2 dual agonist. In some aspects, a composition of this invention, such as an isotonic parenteral pharmaceutical composition of this invention, comprises about 20 mg/mL GLP-1/GLP-2 dual agonist. In some aspects, a composition of this invention, such as an isotonic parenteral pharmaceutical composition of this invention, comprises about 25 mg/mL GLP-1/GLP-2 dual agonist. In some aspects, a composition of this invention, such as an isotonic parenteral pharmaceutical composition of this invention, comprises about 33 mg/mL GLP-1/GLP-2 dual agonist.

Preferably, a composition of this invention comprises from about 2 mg/mL to about 25 mg/mL GLP-1/GLP-2 dual agonist, preferably from about 6 mg/mL to about 25 mg/mL GLP-1/GLP-2 dual agonist. Most preferably, a composition of this invention comprises about 15 mg/mL GLP-1/GLP-2 dual agonist.

***Synthesis of dual agonists***

It is preferred to synthesise dual agonists of the invention by means of solid-phase or liquid-phase peptide synthesis methodology. In this context, reference may be made to WO 98/11 125 and, among many others, Fields, G.B. et al., 2002, "Principles and practice of solid-phase peptide synthesis". In: Synthetic Peptides (2nd Edition), and the Examples herein. In accordance with the present invention, a dual agonist of the invention may be synthesised or produced in a number of ways, including for example, a method which comprises:

(a) synthesising the dual agonist by means of solid-phase or liquid-phase peptide synthesis methodology and recovering the synthesised dual agonist thus obtained; or

(b) expressing a precursor peptide sequence from a nucleic acid construct that encodes the precursor peptide, recovering the expression product, and modifying the precursor peptide to yield a compound of the invention.

The precursor peptide may be modified by introduction of one or more non-proteinogenic amino acids, e.g. Aib, Orn, Dap, or Dab, introduction of an albumin binding moiety or introduction of the appropriate terminal groups -OH or $-NH_2$, etc.

Expression is typically performed from a nucleic acid encoding the precursor peptide, which may be performed in a cell or a cell-free expression system comprising such a nucleic acid.

## Preservatives

The composition of this invention, such as an isotonic parenteral pharmaceutical composition of this invention, comprises one or more preservative. In some aspects, the one or more preservative comprises or is m-cresol and/or phenol. In some aspects, the preservative is m-cresol or phenol. The composition of this invention, such as an isotonic parenteral pharmaceutical composition of this invention, comprises a preservative, wherein the preservative comprises or is m-cresol and/or phenol. In some aspects the composition of this invention, such as an isotonic parenteral pharmaceutical composition of this invention, comprises one preservative, wherein the preservative comprises or is m-cresol or phenol. In some aspects the composition of this invention, such as an isotonic parenteral pharmaceutical composition of this invention, comprises two preservatives, wherein the preservatives comprise or are m-cresol and phenol.

### *m-Cresol*

In some aspects, the composition of this invention, such as an isotonic parenteral pharmaceutical composition of this invention, comprises one or more preservative, wherein the one or more preservative comprises m-cresol. In some aspects, the one or more preservative is m-cresol. In some aspects, the composition of this invention, such as an isotonic parenteral pharmaceutical composition of this invention, comprises a preservative which is m-cresol. In some aspects, the composition of this invention, such as an isotonic parenteral pharmaceutical composition of this invention, comprises m-cresol.

m-Cresol is an organic compound also known as meta-cresol, 3-cresol, 3-methylphenol, 3-methylbenzenol, 3-hydroxytoluene or 1-hydroxy-3-methylbenzene. m-Cresol has the chemical formula $CH_3C_6H_4(OH)$ and the following structural formula:

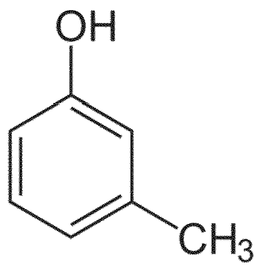

In some aspects, the m-cresol is present in the composition of the invention at a concentration of from about 1.15 mg/mL to about 5.15 mg/mL. In some aspects, the m-cresol is present in the composition of the invention at a concentration of about 1.15 mg/mL. In some aspects, the m-cresol is present in the composition of the invention at a concentration of about 5.15 mg/mL. Preferably, the m-cresol is present in the composition of the invention at a concentration of about 3.15 mg/mL.

### *Phenol*

In some aspects, the composition of this invention, such as an isotonic parenteral pharmaceutical composition of this invention, comprises one or more preservative, wherein the one or more preservative comprises phenol. In some aspects, the one or more preservative is phenol. In some aspects, the composition of this invention, such as an isotonic parenteral pharmaceutical composition of this invention, comprises a preservative which is phenol. In some aspects, the composition of this invention, such as an isotonic parenteral pharmaceutical composition of this invention, comprises phenol.

Phenol is an organic compound also known as benzenol. Phenol has the chemical formula $C_6H_5OH$ and the following structural formula:

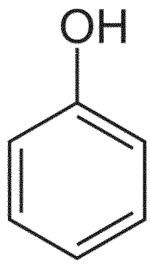

In some aspects, the phenol is present in the composition of the invention at a concentration of from about 2.5 mg/mL to about 8.5 mg/mL. In some aspects, the phenol is present in the composition of the invention at a concentration of about 2.5 mg/mL. In some aspects, the phenol is present in the composition of the invention at a concentration of about 8.5 mg/mL. Preferably, the phenol is present in the composition of the invention at a concentration of about 5.5 mg/mL.

**Tris buffer and pH**

The composition of the invention, such as an isotonic parenteral pharmaceutical composition of the invention, comprises tris buffer. In some aspects, the tris buffer is present in the composition, such as an isotonic parenteral pharmaceutical composition, at a final concentration of from about 5 mM to about 50 mM, such as from about 5 mM to about 20 mM. In some aspects, the tris buffer is present in the composition at a final concentration of about 5 mM. In some aspects, the tris buffer is present in the composition at a final concentration of about 50 mM. Preferably, the tris buffer is present in the composition at a final concentration of about 20 mM.

The term "tris" is short for tris(hydroxymethyl)aminomethane. Tris is also known as 2-amino-2-(hydroxymethyl) propane-1,3-diol, has the chemical formula $(HOCH_2)_3CNH_2$ and the following structural formula:

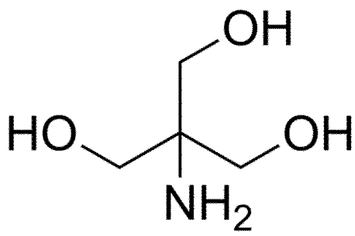

The term "tris buffer" refers to a buffer comprising tris molecules.

In some aspects, the pH of a composition, such as an isotonic parenteral pharmaceutical composition, of this invention, is between about pH 6.0 and about pH 8.5, for example between about pH 6.0 and about pH 8.4, between about pH 6.0 and about pH 8.3, between about pH 6.0 and about pH 8.2, between about pH 6.0 and about pH 8.1 or between about pH 6.0 and about pH 8.0. In one aspect the pH is preferably between about pH 7.0 to about pH 8.0. In some aspects, the pH is a pH of from about pH 6.5 to about pH 8.5. In some aspects, said pH of a composition, such as an isotonic parenteral pharmaceutical composition, of this invention, is between about pH 7.0 to about pH 8.0. In some aspects, said pH of a composition, such as an isotonic parenteral pharmaceutical composition, of this invention, is about pH 7.0. In some aspects, said pH of a composition, such as an isotonic parenteral pharmaceutical composition, of this invention, is about pH 8.0. In some aspects, said pH of a composition of this invention is about pH 8.2. In some aspects, said pH of a composition of this invention is about pH 6.0. In some aspects, said pH of a composition of this invention is between about pH 7.0 and about pH 8.2, preferably about pH 7.5 or about pH 8.2. In some aspects, said pH of a composition of this invention is between about pH 7.0 and about pH 8.2, preferably about pH 7.6 or about 8.0. In some aspects, said pH of a composition of this invention is between about pH 7.0 and about pH 8.2, preferably about pH 7.6 or about pH 7.7. In some aspects, said pH of a composition of this invention is between about pH 7.0 and about pH 8.2, preferably about pH 7.6. In some aspects, said pH of a composition of this invention is between about pH 7.0 and about pH 8.2, preferably about pH 8.0. In some aspects, said pH of a composition of this invention is between about pH 7.0 and about pH 8.2, preferably about pH 7.0. In a preferred aspect the pH is about 8.0.

In some aspects, in the composition of the invention the pH is adjusted with either NaOH or HCl as needed.

**Tonicity and tonicity agents**

In some aspects, the composition of this invention is an isotonic parenteral pharmaceutical composition.

In some aspects, the composition of this invention comprising one or more GLP-1/GLP-2 dual agonist comprising formula A or B is isotonic. In some aspects, the composition of this invention comprising one or more GLP-1/GLP-2 dual agonist comprising SEQ ID NO: 1 is isotonic.

In some embodiments, the osmolality of the compositions of this invention is about 300 $\pm$120 mOsmol/kg. In some embodiments, the osmolality of the compositions of this invention is about 290 $\pm$70 mOsmol/kg. In some embodiments, the osmolality of the compositions of this invention is about 230 mOsmol/kg to about 370 mOsmol/kg. In some embodiments, the osmolality of the compositions of this invention is about 280 mOsmol/kg to about 320 mOsmol/kg. In some embodiments the osmolality of the compositions of this invention is about 290 mOsmol/kg to about 320 mOsmol/kg.

In some aspects, the composition of this invention, such as an isotonic parenteral pharmaceutical composition of

this invention, comprises one or more tonicity agent.

The term "tonicity agent" refers to excipients added to the composition according to the invention in order to achieve isotonicity relative to bodily fluids. A range of ionic and non-ionic tonicity agents are used in pharmaceutical compositions. Non-ionic tonicity agents may be selected from dextrose, propylene glycol, glyceryl, mannitol, such as D-mannitol and sorbitol. Ionic tonicity agents may include, alkali metals or earth metal halides, such as $CaCl_2$, KBr, KCl, LiCl, NaI, NaBr, NaCl, $Na_2SO_4$. In one aspect the tonicity agent may be selected from mannitol, NaCl and propylene glycol.

"Ionic compounds" are two or more ions held together by attraction. An example of an ionic compound is table salt. It consists of positive sodium ions and negative chloride ions. They have high melting and boiling points and are hard or brittle. They can also be dissolved in water. The definition for a "non-ionic compound" is that the chemical bonds in this compound are non-ionic. They usually have chemical bonds that share electron density.

In some aspects, the one or more tonicity agent comprises or is mannitol. Preferably, the one or more tonicity agent is D-mannitol. In some aspects, the mannitol, such as D-mannitol, is present in the composition of the invention at a concentration of from about 130 mM to about 330 mM, preferably from about 150 mM to about 300 mM, more preferably from about 190 mM to about 240 mM. In some aspects, the mannitol, such as D-mannitol, is present in the composition of the invention at about 230 mM.

In some aspects, the one or more tonicity agent comprises or is NaCl. In some aspects, the NaCl is present in the composition of the invention at a concentration of from about 50 mM to about 450 mM, preferably from about 65 mM to about 165 mM. Preferably, the NaCl is present at a concentration of about 125 mM.

The term "isotonic" as used herein, refers to the tonicity relative to body fluids at the site of injection, i.e. *i.v.* or s.c.. Thus, the term "isotonic" is used to describe that the pharmaceutical composition has the same tonicity as body fluids at the injection site, such as red blood cells and/or blood plasma. Compositions with an osmolality of about 300 mOsmol/kg, such as about 280-320 mOsmol/kg or about 290-320 mOsmol/kg are considered as isotonic.

Isotonicity is important for parenteral pharmaceutical compositions, because a "hypotonic" solution causes a cell to swell, whereas a "hypertonic" solution causes a cell to shrink. Although it is related to osmolality, tonicity also takes into consideration the ability of the solute to cross the cell membrane.

## Compositions of the invention

In some aspects, a composition of this invention, such as an isotonic parenteral pharmaceutical composition of this invention, comprises a solvent. In some aspects, the solvent is water.

In some aspects, a composition of this invention, such as an isotonic parenteral pharmaceutical composition of this invention, comprises a tonicity agent and a solvent.

In some aspects, a composition of this invention, such as an isotonic parenteral pharmaceutical composition of this invention, comprises a GLP-1/GLP-2 dual agonist comprising an amino acid sequence of formula A, m-cresol and tris buffer.

In some aspects, a composition of this invention, such as an isotonic parenteral pharmaceutical composition of this invention, comprises a GLP-1/GLP-2 dual agonist comprising an amino acid sequence of formula A, phenol and tris buffer.

In some aspects, a composition of this invention, such as an isotonic parenteral pharmaceutical composition of this invention, comprises a GLP-1/GLP-2 dual agonist comprising an amino acid sequence of formula A, m-cresol, tris buffer, and mannitol, such as D-mannitol.

In some aspects, a composition of this invention, such as an isotonic parenteral pharmaceutical composition of this invention, comprises a GLP-1/GLP-2 dual agonist comprising an amino acid sequence of formula A, phenol, tris buffer, and mannitol, such as D-mannitol.

In some aspects, in a composition of this invention, such as an isotonic parenteral pharmaceutical composition of this invention, the GLP-1/GLP-2 dual agonist comprises an amino acid sequence of formula A, the preservative is m-cresol at a final concentration of from about 1.15 mg/mL to about 5.15 mg/mL, preferably about 3.15 mg/mL, or the preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist comprises an amino acid sequence of formula A, the preservative is m-cresol at a final concentration of from about 1.15 mg/mL to about 5.15 mg/mL, preferably about 3.15 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist comprises an amino acid sequence of formula A, the preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of

this invention, the GLP-1/GLP-2 dual agonist comprises an amino acid sequence of formula A and is at a concentration of from at least 1 mg/mL to about 33 mg/mL, preferably from at least 1 mg/mL to about 25 mg/mL, preferably from at least 6 mg/mL to about 25 mg/mL, the preservative is m-cresol at a final concentration of from about 1.15 mg/mL to about 5.15 mg/mL, preferably about 3.15 mg/mL, or the preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist comprises an amino acid sequence of formula A and is at a concentration of from at least 1 mg/mL to about 33 mg/mL, preferably from at least 1 mg/mL to about 25 mg/mL, preferably from at least 6 mg/mL to about 25 mg/mL, the preservative is m-cresol at a final concentration of from about 1.15 mg/mL to about 5.15 mg/mL, preferably about 3.15 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist comprises an amino acid sequence of formula A and is at a concentration of from at least 1 mg/mL to about 33 mg/mL, preferably from at least 1 mg/mL to about 25 mg/mL, preferably from at least 6 mg/mL to about 25 mg/mL, the preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist comprises an amino acid sequence of formula A and is at a concentration of from at least 1 mg/mL to about 33 mg/mL, preferably from at least 1 mg/mL to about 25 mg/mL, preferably from at least 6 mg/mL to about 25 mg/mL, the preservative is m-cresol at a final concentration of from about 1.15 mg/mL to about 5.15 mg/mL, preferably about 3.15 mg/mL, or the preservative is phenol at a final concentration of from about 2.5 mg/mL to about 8.5 mg/mL, preferably about 5.5 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

In some aspects, in a composition of this invention, such as an isotonic parenteral pharmaceutical composition of this invention, the GLP-1/GLP-2 dual agonist comprises an amino acid sequence of formula A and is at a concentration of from at least 1 mg/mL to about 25 mg/mL, preferably from at least 6 mg/mL to about 25 mg/mL, the preservative is m-cresol at a final concentration of from about 1.15 mg/mL to about 5.15 mg/mL, preferably about 3.15 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

In some aspects, in a composition of this invention, such as an isotonic parenteral pharmaceutical composition of this invention, the GLP-1/GLP-2 dual agonist comprises an amino acid sequence of formula A and is at a concentration of from at least 1 mg/mL to about 33 mg/mL, preferably from at least 1 mg/mL to about 25 mg/mL, preferably from at least 6 mg/mL to about 25 mg/mL, the preservative is phenol at a final concentration of from about 2.5 mg/mL to about 8.5 mg/mL, preferably about 5.5 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, more preferably from about 6 mg/mL to about 25 mg/mL, the preservative is m-cresol at a final concentration of from about 1.15 mg/mL to about 5.15 mg/mL, preferably about 3.15 mg/mL, or the preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, , more preferably from about 6 mg/mL to about 25 mg/mL, the preservative is m-cresol at a final concentration of from about 1.15 mg/mL to about 5.15 mg/mL, preferably about 3.15 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, more preferably from about 6 mg/mL to about 25 mg/mL, the

preservative is phenol at a final concentration of from about 2.5 mg/mL to about 8.5 mg/mL, preferably about 5.5 mg/mL, and the tris buffer is at a final concentration of about 5 mM to about 50 mM, preferably about 20 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, more preferably from about 6 mg/mL to about 25 mg/mL, the preservative is m-cresol at a final concentration of from about 1.15 mg/mL to about 5.15 mg/mL, preferably about 3.15 mg/mL, or the preservative is phenol at a final concentration of from about 2.5 mg/mL to about 8.5 mg/mL, preferably about 5.5 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, , more preferably from about 6 mg/mL to about 25 mg/mL, the preservative is m-cresol at a final concentration of from about 1.15 mg/mL to about 5.15 mg/mL, preferably about 3.15 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

In some aspects, in a composition of this invention, such as an isotonic parenteral pharmaceutical composition of this invention, the GLP-1/GLP-2 dual agonist is CPD1OH or a pharmaceutically acceptable salt thereof, preferably at a final concentration of from about 1 mg/mL to about 33 mg/mL, preferably from about 1 mg/mL to about 25 mg/mL, preferably from about 2 mg/mL to about 25 mg/mL, more preferably from about 6 mg/mL to about 25 mg/mL, the preservative is phenol at a final concentration of from about 2.5 mg/mL to about 8.5 mg/mL, preferably about 5.5 mg/mL, the tris buffer is at a final concentration of about 5 mM to about 50 mM, preferably about 20 mM, and the tonicity agent is mannitol, such as D-mannitol, at a final concentration of from about 130 mM to about 330 mM, preferably from about 150 mM to about 300 mM, preferably from about 190 mM to about 240 mM, preferably about 230 mM.

## Indications

In some aspects, a pharmaceutical composition of this invention is administered to human subjects in the need of prophylaxis or treatment of intestinal damage and dysfunction, regulation of body weight, and prophylaxis or treatment of metabolic dysfunction.

In some aspects, a pharmaceutical composition of this invention is administered to human subjects in the need of prophylaxis or treatment of malabsorption, ulcers (e.g. peptic ulcers, Zollinger-Ellison Syndrome, drug-induced ulcers, and ulcers related to infections or other pathogens), short-bowel syndrome, cul-de-sac syndrome, inflammatory bowel disease (Crohns disease and ulcerative colitis), irritable bowel syndrome (IBS), pouchitis, celiac sprue (for example arising from gluten induced enteropathy or celiac disease), tropical sprue, hypogammaglobulinemic sprue, mucositis induced by chemotherapy or radiation therapy, diarrhoea induced by chemotherapy or radiation therapy, low grade inflammation, metabolic endotoxemia, necrotising enterocolitis, primary biliary cirrhosis, hepatitis, fatty liver disease (including parental nutrition associated gut atrophy, PNALD (Parenteral Nutrition-Associated Liver Disease), NAFLD (Non-Alcoholic Fatty Liver Disease) and NASH (Non-Alcoholic Steatohepatitis)), or gastrointestinal side-effects of inflammatory conditions such as pancreatitis or graft versus host disease (GVHD).

In some aspects, a pharmaceutical composition of this invention is administered to human subjects in the need of prophylaxis or treatment of obesity, morbid obesity, obesity-linked gallbladder disease, obesity-induced sleep apnoea, inadequate glucose control, glucose tolerance, dyslipidaemia (e.g. elevated LDL levels or reduced HDL/LDL ratio), diabetes (e.g. Type 2 diabetes, gestational diabetes), pre-diabetes, metabolic syndrome or hypertension.

In some aspects, a pharmaceutical composition of this invention is administered to human subjects to facilitate biological effects selected from the group consisting of: increasing intestinal mass, improving intestinal function (especially intestinal barrier function), increasing intestinal blood flow, repairing intestinal damage or dysfunction in a subject in need thereof.

In some aspects, a pharmaceutical composition of this invention is administered to human subjects in the need of prophylaxis or treatment of intestinal dysfunction or damage caused by or associated with GVHD, as well as prophylaxis or treatment of side effects such as diarrhoea caused by or associated with GVHD.

In some aspects, a pharmaceutical composition of this invention is administered to human subjects in the need prophylaxis or treatment of obesity, morbid obesity, obesity-linked gallbladder disease and obesity-induced sleep apnoea.

In some aspects, a pharmaceutical composition of this invention is administered to human subjects in the need of improving glucose tolerance and/or glucose control. In some aspects, a pharmaceutical composition of this invention is

administered to human subjects in the need of modulating (e.g. improving) circulating cholesterol levels, being capable of lowering circulating triglyceride or LDL levels, and increasing HDL/LDL ratio.

## Administration

In some aspects, a pharmaceutical composition of this invention is an aqueous composition. In some aspects, a pharmaceutical composition of this invention is suitable for parenteral administration performed by subcutaneous (*s.c.*), intramuscular (*i.m.*) or intravenous (*i.v.*) injection by means of a syringe, optionally a pen-like syringe. In some aspects, a pharmaceutical composition of this invention is suitable for *s.c.* injection into human subjects. In some aspects, a pharmaceutical composition of this invention is suitable for *i.v.* injection into human subjects.

In some aspects, the isotonic parenteral pharmaceutical composition of this invention is suitable for a single dose administration. In some aspects the isotonic parenteral pharmaceutical composition of is injection is suitable for injection in a single use device. In some aspects, the single use device is selected from an injector pen or single use syringe. In some aspects, the isotonic parenteral pharmaceutical composition of this invention is suitable for a multi dose administration.

In some aspects the isotonic parenteral pharmaceutical composition of this invention is administered by s.c. injection into human subjects in a volume allowing for a total amount of from about 1 mg to about 25 mg of GLP-1/GLP-2 dual agonist to be delivered to the subject. In some aspects the isotonic parenteral pharmaceutical composition of this invention is administered by *s.c.* injection into human subjects in a volume allowing for a total amount of about 1 mg or more of GLP-1/GLP-2 dual agonist to be delivered to the subject. In some aspects the isotonic parenteral pharmaceutical composition of this invention is administered by s.c. injection into human subjects in a volume allowing for a total amount of about 2 mg to about 25 mg of GLP-1/GLP-2 dual agonist to be delivered to the subject, preferably in a volume allowing for a total amount of about 6 mg to about 25 mg of GLP-1/GLP-2 dual agonist to be delivered to the subject. In some aspects the isotonic parenteral pharmaceutical composition of this invention is administered by *s.c.* injection into human subjects in a volume allowing for a total amount of about 1 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 2 mg or more of GLP-1/GLP-2 dual agonist to be delivered to the subject. In some aspects the isotonic parenteral pharmaceutical composition of this invention is administered by s.c. injection into human subjects in a volume allowing for a total amount of about 3 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 4 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 5 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 6 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 9 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 10 mg or more of GLP-1/GLP-2 dual agonist to be delivered to the subject. In some aspects the isotonic parenteral pharmaceutical composition of this invention is administered by *s.c.* injection into human subjects in a volume allowing for a total amount of about 7, 8, 11, 12, 13, 14, 15, 16, 17, 18, 19, 20, 21, 22, 23, 24, or 25 mg or more of GLP-1/GLP-2 dual agonist to be delivered to the subject.

## Biological activity

In some aspects, peptides comprised in pharmaceutical compositions of this invention are peptides according to formula A and SEQ ID NO: 1 which have previously been described in patent application WO2018104561, which describes the compounds, their preparation and purification and biologic activity (Table 5, WO2018104561). Example 2 in WO2018104561 includes data on *in vitro* potency on the GLP-1 and GLP-2 receptor.

## Chemical stability (purity)

The compositions of this invention, such as isotonic parenteral pharmaceutical compositions of this invention, provide good chemical stability. In other words, in compositions of this invention, the GLP-1/GLP-2 dual agonist remains chemically stable during storage. Chemical stability may be comparable relative to an equivalent composition which does not comprise the preservative according to the invention as described herein.

In some aspects, the composition of this invention has good relative purity. Relative purity may be comparable or improved relative to an equivalent composition which does not comprise the preservative according to the invention as

described herein.

Where the "chemical stability" of a composition of this invention is referred to herein, this means the chemical stability of the GLP-1/GLP-2 dual agonist comprised in the composition. In some aspects, chemical stability of the GLP-1/GLP-2 dual agonist is determined using ASSAY I described herein.

The chemical stability at time point Y of the GLP-1/GLP-2 dual agonist in any of the tested compositions disclosed herein can be expressed as the relative purity $X^Y$ of the GLP-1/GLP-2 dual agonist and is determined by measuring the absolute purity X' of the GLP-1/GLP-2 dual agonist and normalising it to the absolute purity X° of the GLP-1/GLP-2 dual agonist at day zero (day 0), wherein said absolute purities are determined by HPLC at a given time point Y by identifying the purity of peak corresponding to the GLP-1/GLP-2 dual agonist.

Thus, at day zero (day 0), the absolute purity X' is the same as the absolute purity X° and thus chemical stability of a GLP-1/GLP-2 dual agonist in the tested composition, expressed as the relative purity $X^Y$= 100%, wherein Y= day 0.

Relative purity can be calculated the following way:

$$X^Y = (X'/X^0)*100$$

wherein X is the relative purity at a given time point Y, X° is the absolute purity on day 0 and X' is the absolute purity on the given time point Y,

wherein the absolute purity X° or X' of the GLP-1/GLP-2 dual agonist in the tested composition are determined by HPLC, identifying the purity of peak corresponding to the GLP-1/GLP-2 dual agonist.

Relative purity at a given time point may be calculated by multiplying the purity slope by the number of weeks of storage, and subtracting the modulus of this value from 100%.

In some aspects, the pharmaceutical compositions of this invention lead to a relative purity of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 2 weeks storage, for example at 40°C. In some aspects, the pharmaceutical compositions of this invention lead to a relative purity of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 26 weeks storage, for example at 25°C. In some aspects, the pharmaceutical compositions of this invention lead to a relative purity of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 52 weeks storage, for example at 5°C.

## Chemical stability: Oligomerisation

Peptides in solution can aggregate to form therapeutically inactive covalently linked oligomers. Peptide oligomerisation may be measured using size exclusion chromatography (SEC), such as described in ASSAY II herein.

Various analytical techniques for measuring peptide stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10: 29-90 (1993), for example.

It was surprisingly found that the peptide of the invention is more stable and oligomerises less when m-cresol preservative is combined with tris buffer rather than phosphate buffer.

Relative total non-oligomerised peptide at a given time point may be calculated by multiplying the oligomer slope by the number of weeks of storage, and subtracting the modulus of this value from 100%.

In some aspects, the pharmaceutical compositions of this invention lead to a relative total non-oligomerised peptide (monomer) of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 2 weeks storage, for example at 40°C. In some aspects, the pharmaceutical compositions of this invention lead to a relative total non-oligomerised peptide of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 26 weeks storage, for example at 25°C. In some aspects, the pharmaceutical compositions of this invention lead to a relative total non-oligomerised peptide of said one or more GLP-1/GLP-2 dual agonist, such as CPD1 or any pharmaceutically acceptable salt thereof, of at least about 85%, 86%, 87%, 88%, 89%, 90%, 91%, 92%, 93%, 94%, 95%, 96%, 97%, 98% or 99%, for example after at least 52 weeks storage, for example at 5°C.

## LIST OF ABBREVIATIONS

| Abbreviation | Explanation |
|---|---|
| *i.v.* | Intravenous |
| *s.c.* | Subcutaneous |
| HPLC | High-performance liquid chromatography |
| NO | Not determined |
| N.P. | No particulates |
| part/cont | Particles per container |
| RP-HPLC | Reverse Phase High-performance liquid chromatography |
| CPD | Compound |
| SEQ ID NO | Sequence identification number |

## TERMS & DEFINITIONS

When using terms such as "about" and "approximately" in relation to numerical values, the skilled person should immediately recognise that any effect or result, which may be associated with the given values can be obtained within a certain tolerance from the particular values. The term "about" as used herein thus means in reasonable vicinity of the stated numerical value, such as plus or minus 10%. When the term "about" is used about the chemical stability in this patent application, the reasonable vicinity will be below 2%, such as 0.5% or 0.75%, 1% or 1.5%.

The term "solvent" as used herein is meant to be a substance that dissolves a solute (a chemically distinct liquid, solid or gas), resulting in a solution. A solvent is usually a liquid but can also be a solid, a gas, or a supercritical fluid. Solvents are generally classified by the polarity, and considered either polar or non-polar, as indicated by the dielectric constant. Generally, solvents with dielectric constants greater than about 5 are considered "polar" and those with dielectric constants less than 5 are considered "non-polar".

## NON-LIMITING ASPECTS OF THE INVENTION

The present invention is defined by the claims. Any "embodiment", "'aspect" or "example" which is disclosed in the description but is not covered by the claims should be considered as presented for illustrative purpose only.

1. A composition comprising:

(a) one or more GLP-1/GLP-2 dual agonist comprising general formula A:

H[Aib]EG-X5-F-X7-SELATILD-[Ψ]-QAARDFIAWLI-X28-HKITD (A),

wherein X5 is T or S; X7 is T or S; X28 is Q, E, A, H, Y, L, K, R or S and at least one of X5 and X7 is T, wherein [Ψ] indicates an L or D lysine residue in which an albumin binding moiety is conjugated to the GLP-1/GLP-2 dual agonist, and
wherein said albumin binding moiety is [K([17-carboxy-heptadecanoyl]-isoGlu)];

(b) one or more preservative, wherein the one or more preservative comprises or is m-cresol and/or phenol; and
(c) tris buffer.

2. The composition according to aspect 1, wherein the composition is an isotonic parenteral pharmaceutical composition.

3. The composition according to any one of the preceding aspects, wherein the one or more preservative comprises or is m-cresol, preferably wherein the m-cresol is present at a concentration of from about 1.15 mg/mL to about 5.15 mg/mL, more preferably wherein the m-cresol is present at a concentration of about 3.15 mg/mL.

4. The composition according to any one of the preceding aspects, wherein the one or more preservative comprises or is phenol, preferably wherein the phenol is present at a concentration of from about 2.5 mg/mL to about 8.5 mg/mL, more preferably wherein the phenol is present at a concentration of about 5.5 mg/mL.

5. The composition according to any one of the preceding aspects, wherein the tris buffer is present at a concentration of from about 5 mM to about 50 mM, preferably wherein the tris buffer is present at a concentration of about 20 mM.

6. The composition according to any one of the preceding aspects, wherein the composition has a pH of from about pH 6.0 to about pH 8.5, preferably a pH of from about pH 6.5 to about pH 8.5, preferably a pH of from about pH 7.0 to about pH 8.0, more preferably a pH of about pH 8.0.

7. The composition according to any one of the preceding aspects, wherein the one or more GLP-1/GLP-2 dual agonist is of the general formula B:

H[Aib]EG-X5-FT-SELATILD-[Ψ]-QAARDFIAWLI-X28-HKITD (B),

wherein X5 is T or S; X28 is Q, E, A, H, Y, L, K, R or S and
wherein [Ψ] indicates an L or D lysine residue in which the albumin binding moiety is conjugated to the GLP-1/GLP-2 dual agonist and
wherein said albumin binding moiety is [K([17-carboxy-heptadecanoyl]-isoGlu)].

8. The composition according any one of the preceding aspects, wherein the one or more GLP-1/GLP-2 dual agonist comprises the sequence:
H[Aib]EGSFTSELATILD[Ψ]QAARDFIAWLIQHKITD (SEQ ID NO: 1).

9. The composition according to any one of the preceding aspects, wherein the one or more GLP-1/GLP-2 dual agonist is:

Hy-H[Aib]EGSFTSELATILD[K([17-carboxy-heptadecanoyl]-isoGlu)]QAARDFIAWLIQHKITD-OH (CPD1OH);

or

Hy-H[Aib]EGSFTSELATILD[K([17-carboxy-heptadecanoyl]-isoGlu)]QAARDFIAWLIQHKITD-$NH_2$ (CPD1$NH_2$),

or a pharmaceutically acceptable salt of CPD1OH or CPD1$NH_2$, preferably a chloride salt of CPD1OH or CPD1$NH_2$.

10. The composition according to any one of the preceding aspects, wherein the GLP-1/GLP-2 dual agonist is present at a concentration of at least about 1 mg/mL, preferably a concentration of from about 1 mg/mL to about 33 mg/mL, such as from about 2 mg/mL to about 33 mg/mL, such as a concentration of from about 1 mg/mL to about 25 mg/mL, such as a concentration of from about 6 mg/mL to about 25 mg/mL.

11. The composition according to aspect 11, wherein the GLP-1/GLP-2 dual agonist is present at a concentration of about 2 mg/mL, about 15 mg/mL or about 25 mg/mL.

12. The composition according to any one of the preceding aspects, wherein the composition further comprises one or more tonicity agent.

13. The composition according to aspect 12, wherein the one or more tonicity agent comprises or is mannitol, preferably D-mannitol.

14. The composition according to aspect 13, wherein the mannitol is present at a concentration of from about 130 mM to about 330 mM, preferably from about 150 mM to about 300 mM, preferably from about 190 mM to about 240 mM, preferably about 230 mM.

15. The composition according to aspect 12, wherein the one or more tonicity agent comprises or is NaCl, preferably

wherein the NaCl is present at a concentration of from about 50 mM to about 450 mM, preferably from about 65 mM to about 165 mM preferably about 125 mM.

16. The composition according to any one of the preceding aspects, wherein the osmolality of the composition is about 230 mOsmol/kg to about 370 mOsmol/kg.

17. The composition according to any one of the preceding aspects, wherein the composition further comprises a solvent, preferably water.

18. The composition according to any one of the preceding aspects, wherein the one or more preservative is m-cresol at a concentration of from about 1.15 mg/mL to about 5.15 mg/mL, preferably about 3.15 mg/mL, wherein the tris buffer is at a concentration of about 5 mM to about 50 mM, preferably about 20 mM, wherein the composition further comprises mannitol, preferably D-mannitol, at a concentration of from about 130 mM to about 330 mM, preferably from about 150 mM to about 300 mM, preferably from about 190 mM to about 240 mM, preferably about 230 mM, and wherein the pH of the composition is from about pH 7.0 to about pH 8.0, preferably about pH 8.0.

19. The composition according to any one of the preceding aspects, wherein the one or more preservative is phenol at a concentration of from about 2.5 mg/mL to about 8.5 mg/mL, preferably about 5.5 mg/mL, wherein the tris buffer is at a concentration of about 5 mM to about 50 mM, preferably about 20 mM, wherein the composition further comprises mannitol, preferably D-mannitol, at a concentration of from about 130 mM to about 330 mM, preferably from about 150 mM to about 300 mM, preferably from about 190 mM to about 240 mM, preferably about 230 mM, and wherein the pH of the composition is from about pH 7.0 to about pH 8.0, preferably about pH 8.0.

20. The composition according to any one of the preceding aspects, wherein the one or more GLP-1/GLP-2 dual agonist is CPD1OH or $CPD1NH_2$, preferably CPD1OH, or a chloride salt of CPD1OH or $CPD1NH_2$, preferably a chloride salt of CPD1OH, wherein the one or more preservative is m-cresol at a concentration of from about 1.15 mg/mL to about 5.15 mg/mL, preferably about 3.15 mg/mL, wherein the tris buffer is at a concentration of about 5 mM to about 50 mM, preferably about 20 mM, and wherein the composition further comprises mannitol, preferably D-mannitol, at a concentration of about 230 mM, water, and sodium hydroxide and/or hydrochloric acid for pH adjustment to a pH of about pH 8.0.

21. The composition according to any one of the preceding aspects, wherein the one or more GLP-1/GLP-2 dual agonist is CPD1OH or $CPD1NH_2$, preferably CPD1OH, or a chloride salt of CPD1OH or $CPD1NH_2$, preferably a chloride salt of CPD1OH, wherein the one or more preservative is phenol at a concentration of from about 2.5 mg/mL to about 8.5 mg/mL, preferably about 5.5 mg/mL, wherein the tris buffer is at a concentration of about 5 mM to about 50 mM, preferably about 20 mM, and wherein the composition further comprises mannitol, preferably D-mannitol, at a concentration of about 230 mM, water, and sodium hydroxide and/or hydrochloric acid for pH adjustment to a pH of about pH 8.0.

22. The composition according to any one of the preceding aspects, wherein the composition is suitable for subcutaneous (s.c.) or intravenous (i.v.) injection into human subjects.

23. The composition according to any one of the preceding aspects, for use in:

(i) increasing intestinal mass, improving intestinal function, increasing intestinal blood flow, or repairing intestinal damage or dysfunction, in a subject in need thereof; or
(ii) the prophylaxis or treatment of malabsorption, ulcers, short-bowel syndrome, cul-de-sac syndrome, inflammatory bowel disease, irritable bowel syndrome, pouchitis, celiac sprue, tropical sprue, hypogammaglobulinemic sprue, mucositis induced by chemotherapy or radiation therapy, diarrhoea induced by chemotherapy or radiation therapy, low grade inflammation, metabolic endotoxemia, necrotising enterocolitis, primary biliary cirrhosis, hepatitis, fatty liver disease, or gastrointestinal side-effects of inflammatory conditions, in a subject in need thereof; or
(iii) reducing or inhibiting weight gain, reducing gastric emptying or intestinal transit, reducing food intake, reducing appetite, or promoting weight loss, in a subject in need thereof; or
(iv) the prophylaxis or treatment of obesity, morbid obesity, obesity-linked gallbladder disease, obesity-induced sleep apnoea, inadequate glucose control, glucose tolerance, dyslipidaemia, diabetes, pre-diabetes, metabolic syndrome or hypertension, in a subject in need thereof.

24. A method for preserving a composition comprising one or more GLP-1/GLP-2 dual agonist comprising general formula A:

H[Aib]EG-X5-F-X7-SELATILD-[Ψ]-QAARDFIAWLI-X28-X29-KITD (A),

wherein X5 is T or S; X7 is T or S; X28 is Q, E, A, H, Y, L, K, R or S; X29 is H and at least one of X5 and X7 is T, and wherein [Ψ] indicates an L or D lysine residue in which an albumin binding moiety is conjugated to the GLP-1/GLP-2 dual agonist, and
wherein the albumin binding moiety is [K([17-carboxy-heptadecanoyl]-isoGlu)];
wherein the composition comprises tris buffer; and
wherein the method comprises adding one or more preservative to the composition, wherein the wherein the one or more preservative comprises or is m-cresol and/or phenol.

25. Use of a preservative for preserving a composition comprising one or more GLP-1/GLP-2 dual agonist comprising general formula A:

H[Aib]EG-X5-F-X7-SELATILD-[Ψ]-QAARDFIAWLI-X28-HKITD (A),

wherein X5 is T or S; X7 is T or S; X28 is Q, E, A, H, Y, L, K, R or S and at least one of X5 and X7 is T, and wherein [Ψ] indicates an L or D lysine residue in which an albumin binding moiety is conjugated to the GLP-1/GLP-2 dual agonist, and
wherein said albumin binding moiety is [K([17-carboxy-heptadecanoyl]-isoGlu)];
wherein the composition comprises tris buffer; and
wherein the preservative comprises or is m-cresol and/or phenol.

## GENERAL METHODS USED

## METHOD I - Methods for preparation of GLP-1/GLP-2 dual agonists

The GLP-1/GLP-2 dual agonists were synthesised as described in Example 1 and under General Peptide Synthesis in patent application WO2018/104561.

CPD1 (corresponding to compound 18 in WO2018/104561) was synthesised using a Solid Phase Peptide Synthesis (SPPS) approach and standard Fmoc coupling methodologies. After completed synthesis, the peptide sequence was deprotected and cleaved from the solid support, and the crude peptide was purified using preparative reverse phase HPLC. The peptide was converted to an acceptable salt form (HCl, acetate or Na) and lyophilised to provide the final CPD1 drug substance.

## METHOD II - Method for preparation & analysis of pharmaceutical compositions

### Sample solutions for laboratory scale

The GLP-1/GLP-2 dual agonist drug substance (CPD1) was prepared according to METHOD I and dissolved in MilliQ water (MQW) to give a stock solution of 40 mg/mL active pharmaceutical ingredient (API). pH was measured. This was followed by addition and mixing of the ingredients as illustrated in Tables 5-10 and pH was then adjusted using 1 M NaOH / HCl as needed to reach the appropriate pH. The final concentrations were 2 or 25 mg/mL of CPD1 as indicated in the tables and examples in this application. The laboratory scale compositions were prepared in volumes between 0.5 to about 2 mL.

For stability testing, the samples were stored at 40 °C for 2 weeks in a dark room (i.e. lights switched off). Samples were analyzed by RP-HPLC and SEC-HPLC according to ASSAY I and II respectively.

## ASSAY I - Measuring GLP-1/GLP-2 dual agonist purity & determining the normalised GLP-1/GLP-2 dual agonist purity in % using RP-HPLC

The chemical stability of a GLP-1/GLP-2 dual agonist (peptide) is herein expressed as the relative purity of the peptide peak (i.e. the main peptide peak) determined by HPLC at a given time point, and normalised to the absolute purity of the peptide peak (i.e. main peptide peak) at day zero (T=0), which is set to 100% normalised purity. The RP-HPLC

method is capable of detecting CPD1 degradation products (deamidation, isomerization, hydrolysis and racemization). The RP-HPLC method is not able to detect covalent oligomers, where two or more CPD1 molecules are linked together through a covalent chemical bond - see ASSAY II for more information on detection of covalent oligomers.

The chemical stability of a GLP-1/GLP-2 dual agonist (peptide) prepared according to METHOD I comprised in a parenteral pharmaceutical composition as prepared according to METHOD II are analysed according to the following method:

A Dionex Ultimate 3000 HPLC system (Thermo Fisher), giving a linear gradient, at a flow rate of 0.5 mL/min was used for the analysis. The mobile phase components consisted of 0.3% trifluoroacetic acid (TFA) in 90% acetonitrile / 10% MQW and 0.3% TFA in MQW. A wavelength of 215 nm was used for detection. Injection amount was 2 μg of peptide. The column used for HPLC analysis was a Phenomenex Kinetex C18, 150 by 3.0 mm, 2.6 μm particle size. Runtime was 25 minutes.

***Table 4* - *RP-HPLC method details***

| | |
|---|---|
| Method file name | LP401.073.02 |
| Column | Phenomenex Kinetex C18, 150 by 3.0 mm, 2.6 μm |
| Gradient (time; % B) | 0;40, 20;70, 20.01;95, 22;95, 22.01;40, 25;40 |
| Eluent A | 0.3% TFA in MQW |
| Eluent B | 0.3% TFA in MeCN |
| Flow Rate | 0.500 mL/min |
| Injection Amount | 2 μg |
| Column Temperature | 25 °C |
| Auto Sampler Temp. | 4 °C |
| UV detection | 215 nm |

The results are shown in Tables 5-7 as the degradation slope calculated from the normalized purity results. The slope is a measure for how fast CPD1 degrades. A lower number (i.e. further from 0) represents higher degradation.

**ASSAY II - Method for evaluation of covalent oligomers by Size Exclusion Chromatography (SEC)**

Size Exclusion Chromatography (SEC) experiments were carried out on a Dionex Ultimate 3000 HPLC system (Thermo Fisher), using isocratic elution with a flow rate of 0.5 mL/min. The mobile phase consisted of 0.1% TFA, 45% acetonitrile in MQW. A wavelength of 215 nm was used for detection. Injection amount was 2 μg of peptide. The column used for SEC analysis was a TSKge! SuperSW2000 (TOSOH Corporation), 4μm, 30 x 4.6 mm and the column temperature was 25°C. Runtime was 12 minutes.

The SEC method is able to detect covalent oligomers, where two or more CPD1 molecules are linked together through a covalent chemical bond.

The covalent oligomer data is presented in Tables 5-7. The data is presented as the slope calculated from the covalent oligomer results. The slope is a measure for how fast CPD1 form covalent oligomers. A higher number represents higher covalent oligomer formation.

**EXAMPLES**

These examples investigate the chemical stability and oligomerisation of CPD1 in compositions comprising different preservatives and buffers, stored at different temperatures, for different lengths of time.

CPD1 was produced according to METHOD I. Pharmaceutical compositions (i.e. Formulations) comprising different preservatives were prepared and stored according to METHOD II. The peptide is CPD1OH, which is comprised of the amino acid sequence of formula A. CPD1OH may be interchangeable with $CPD1NH_2$.

Chemical stability of CPD1 is expressed as the slope of the relative purity of the peptide over time. The slope was determined by measuring absolute purity of the peptide peak (i.e. the main CPD1 peak) at each time point using HPLC as described in ASSAY I, then normalising this value to the absolute purity of the peptide peak at T=0 (which was set to 100% purity) to give percentage relative purity at each time point. The slope over the full time-course was calculated from these relative purity values.

Oligomerisation of CPD1 is expressed as the slope of the proportion of covalent oligomers over time, as determined at each time point using ASSAY II.

**Example 1 - Chemical stability of CPD1 in compositions comprising different preservatives**

In this example, compositions comprising m-cresol or phenol preservative (Formulations 1-25) were stored at 40 °C for 2 weeks. The compositions of Formulations 1-25 are shown in Tables 5-7, along with the chemical stability (purity) slope and oligomerisation slope.

***Table 5 - m-cresol preservative with tris buffer***

| Formulation # | Peptide Conc. [mg/mL] | pH | Tris [mM] | Mannitol [mM] | m-cresol [mg/mL] | Normalised purity slope [%/week] (2 weeks, 40 °C) | Oligomer slope [%/week] (2 weeks, 40 °C) |
|---|---|---|---|---|---|---|---|
| 1 | 2 | 8 | 5 | 230 | 1.15 | -1.4 | 0.4 |
| 2 | 2 | 8 | 5 | 230 | 5.15 | -1.4 | 0.6 |
| 3 | 2 | 8 | 50 | 230 | 1.15 | -1.9 | 0.4 |
| 4 | 2 | 8 | 50 | 230 | 5.15 | -2.0 | 0.6 |
| 5 | 25 | 8 | 5 | 230 | 1.15 | -1.3 | 0.3 |
| 6 | 25 | 8 | 5 | 230 | 5.15 | -1.6 | 0.3 |
| 7 | 25 | 8 | 50 | 230 | 1.15 | -1.8 | 0.3 |
| 8 | 25 | 8 | 50 | 230 | 5.15 | -2.0 | 0.3 |

***Table 6 - phenol preservative with tris buffer***

| Formulation # | Peptide Conc. [mg/mL] | pH | Tris [mM] | Mannitol [mM] | phenol [mg/mL] | Normalised purity slope [%/week] (2 weeks, 40 °C) | Oligomer slope [%/week] (2 weeks, 40 °C) |
|---|---|---|---|---|---|---|---|
| 9 | 2 | 8 | 5 | 230 | 2.5 | -1.5 | 0.6 |
| 10 | 2 | 8 | 5 | 230 | 8.5 | -2.2 | 0.9 |
| 11 | 2 | 8 | 50 | 230 | 2.5 | -2.1 | 0.6 |
| 12 | 2 | 8 | 50 | 230 | 8.5 | -2.5 | 0.9 |
| 13 | 25 | 8 | 5 | 230 | 2.5 | -1.8 | 0.5 |
| 14 | 25 | 8 | 5 | 230 | 8.5 | -1.8 | 0.6 |
| 15 | 25 | 8 | 50 | 230 | 2.5 | -2.0 | 0.4 |
| 16 | 25 | 8 | 50 | 230 | 8.5 | -3.0 | 0.9 |

***Table 7 - m-cresol or phenol preservative with phosphate buffer***

| Formulation # | Peptide conc. (mg/mL) | pH | phosphate (mM) | mannitol (mM) | m-cresol (mg/mL) | phenol (mg/mL) | Normalised purity slope [%/week] (2 weeks, 40 °C) | Oligomer slope [%/week] (2 weeks, 40 °C) |
|---|---|---|---|---|---|---|---|---|
| **17** | 2 | 8 | 20 | 230 | 3.15 | - | -2.3 | 1.5 |
| **18** | 2 | 8 | 5 | 230 | 1.15 | - | -1.3 | 0.5 |
| **19** | 2 | 8 | 50 | 230 | 1.15 | - | -1.9 | 0.6 |
| **20** | 2 | 8 | 5 | 230 | 5.15 | - | -1.7 | 1.0 |
| **21** | 2 | 8 | 50 | 230 | 5.15 | - | -2.5 | 1.5 |
| **22** | 2 | 8 | 5 | 230 | - | 2.5 | 1.6 | 0.4 |
| **23** | 2 | 8 | 50 | 230 | - | 2.5 | -1.9 | 0.5 |
| **24** | 2 | 8 | 5 | 230 | - | 8.5 | -1.7 | 0.8 |

(continued)

| Formulation # | Peptide conc. (mg/mL) | pH | phosphate (mM) | mannitol (mM) | m-cresol (mg/mL) | phenol (mg/mL) | Normalised purity slope [%/week] (2 weeks, 40 °C) | Oligomer slope [%/week] (2 weeks, 40 °C) |
|---|---|---|---|---|---|---|---|---|
| **25** | 2 | 8 | 50 | 230 | - | 8.5 | -2.2 | 1.0 |

In general, the data show that high peptide concentration decreases the amount of covalent oligomers in the presence of the preservatives m-cresol and phenol compared to low peptide concentrations. High peptide concentration either has no effect on or improves stability (as expressed by the purity slope).

Furthermore, the data show that the peptide is more stable and oligomerises less when m-cresol preservative is combined with tris buffer rather than phosphate buffer.

## Claims

**1.** A composition comprising:

(a) one or more GLP-1/GLP-2 dual agonist comprising general formula A:

H[Aib]EG-X5-F-X7-SELATILD-[Ψ]-QAARDFIAWLI-X28-HKITD (A),

wherein X5 is T or S; X7 is T or S; X28 is Q, E, A, H, Y, L, K, R or S and at least one of X5 and X7 is T, wherein [Ψ] indicates an L or D lysine residue in which an albumin binding moiety is conjugated to the GLP-1/GLP-2 dual agonist, and
wherein said albumin binding moiety is [K([17-carboxy-heptadecanoyl]-isoGlu)];

(b) one or more preservative, wherein the one or more preservative comprises or is m-cresol and/or phenol; and
(c) tris buffer.

**2.** The composition according to claim 1, wherein the composition is an isotonic parenteral pharmaceutical composition.

**3.** The composition according to any one of the preceding claims, wherein the one or more preservative comprises or is m-cresol, preferably wherein the m-cresol is present at a concentration of from 1.15 mg/mL to 5.15 mg/mL, more preferably wherein the m-cresol is present at a concentration of 3.15 mg/mL.

**4.** The composition according to any one of the preceding claims, wherein the one or more preservative comprises or is phenol, preferably wherein the phenol is present at a concentration of from 2.5 mg/mL to 8.5 mg/mL, more preferably wherein the phenol is present at a concentration of 5.5 mg/mL.

**5.** The composition according to any one of the preceding claims, wherein the tris buffer is present at a concentration of from 5 mM to 50 mM, preferably wherein the tris buffer is present at a concentration of 20 mM.

**6.** The composition according to any one of the preceding claims, wherein the composition has a pH of from pH 6.0 to pH 8.5, preferably a pH of from pH 6.5 to pH 8.5, preferably a pH of from pH 7.0 to pH 8.0, more preferably a pH of pH 8.0.

**7.** The composition according to any one of the preceding claims, wherein the one or more GLP-1/GLP-2 dual agonist is of the general formula B:

H[Aib]EG-X5-FT-SELATILD-[Ψ]-QAARDFIAWLI-X28-HKITD (B),

wherein X5 is T or S; X28 is Q, E, A, H, Y, L, K, R or S and
wherein [Ψ] indicates an L or D lysine residue in which the albumin binding moiety is conjugated to the GLP-1/GLP-2 dual agonist and
wherein said albumin binding moiety is [K([17-carboxy-heptadecanoyl]-isoGlu)].

**8.** The composition according any one of the preceding claims, wherein the one or more GLP-1/GLP-2 dual agonist comprises the sequence:
H[Aib]EGSFTSELATILD[Ψ]QAARDFIAWLIQHKITD (SEQ ID NO: 1).

**9.** The composition according to any one of the preceding claims, wherein the one or more GLP-1/GLP-2 dual agonist is:

Hy-H[Aib]EGSFTSELATILD[K([17-carboxy-heptadecanoyl]-isoGlu)]QAARDFIAWLIQHKITD-OH (CPD1OH);

or

Hy-H[Aib]EGSFTSELATILD[K([17-carboxy-heptadecanoyl]-isoGlu)]QAARDFIAWLIQHKITD-$NH_2$ (CPD1$NH_2$),

or a pharmaceutically acceptable salt of CPD1OH or CPD1$NH_2$, preferably a chloride salt of CPD1OH or CPD1$NH_2$.

**10.** The composition according to any one of the preceding claims, wherein the GLP-1/GLP-2 dual agonist is present at a concentration of at least 1 mg/mL, preferably a concentration of from 1 mg/mL to 33 mg/mL, such as a concentration of from 2 mg/mL to 33 mg/mL, such as a concentration of from 1 mg/mL to 25 mg/mL, such as a concentration of from 6 mg/mL to 25 mg/mL.

**11.** The composition according to claim 10, wherein the GLP-1/GLP-2 dual agonist is present at a concentration of 2 mg/mL, 15 mg/mL or 25 mg/mL.

**12.** The composition according to any one of the preceding claims, wherein the composition further comprises one or more tonicity agent.

**13.** The composition according to claim 12, wherein the one or more tonicity agent comprises or is mannitol, preferably D-mannitol.

**14.** The composition according to claim 13, wherein the mannitol is present at a concentration of from 130 mM to 330 mM, preferably from 150 mM to 300 mM, preferably from 190 mM to 240 mM, preferably 230 mM.

**15.** The composition according to claim 12, wherein the one or more tonicity agent comprises or is NaCl, preferably wherein the NaCl is present at a concentration of from 50 mM to 450 mM, preferably from 65 mM to 165 mM, preferably 125 mM.

## Patentansprüche

**1.** Zusammensetzung, umfassend:

(a) einen oder mehrere GLP-1/GLP-2-Dualagonisten, umfassend die allgemeine Formel A:

H[Aib]EG-X5-F-X7-SELATILD-[Ψ]-QAARDFIAWLI-X28-HKITD (A),

wobei X5 T oder S ist; X7 T oder S ist; X28 Q, E, A, H, Y, L, K, R oder S ist und mindestens eines von X5 und X7 T ist,
wobei [Ψ] einen L- oder D-Lysinrest andeutet, in dem ein Albuminbindungsmolekülteil an den GLP-1/GLP-2-Dualagonisten konjugiert ist, und
wobei der Albuminbindungsmolekülteil [K([17-carboxy-heptadecanoyl]-isoGlu)] ist;

(b) einen oder mehrere Konservierungsstoffe, wobei der eine oder die mehreren Konservierungsstoffe m-Cresol und/oder Phenol umfassen oder sind; und
(c) Tris-Puffer.

2. Zusammensetzung nach Anspruch 1, wobei die Zusammensetzung eine isotonische parenterale pharmazeutische Zusammensetzung ist.

3. Zusammensetzung nach einem der vorhergehenden Ansprüche, wobei der eine oder die mehreren Konservierungsstoffe m-Cresol umfassen oder sind, vorzugsweise wobei das m-Cresol in einer Konzentration von 1,15 mg/ml bis 5,15 mg/ml vorliegt, stärker bevorzugt wobei das m-Cresol in einer Konzentration von 3,15 mg/ml vorliegt.

4. Zusammensetzung nach einem der vorhergehenden Ansprüche, wobei der eine oder die mehreren Konservierungsstoffe Phenol umfassen oder sind, vorzugsweise wobei das Phenol in einer Konzentration von 2,5 mg/ml bis 8,5 mg/ml vorliegt, stärker bevorzugt, wobei das Phenol in einer Konzentration von 5,5 mg/ml vorliegt.

5. Zusammensetzung nach einem der vorhergehenden Ansprüche, wobei der Tris-Puffer in einer Konzentration von 5 mM bis 50 mM vorliegt, vorzugsweise wobei der Tris-Puffer in einer Konzentration von 20 mM vorliegt.

6. Zusammensetzung nach einem der vorhergehenden Ansprüche, wobei die Zusammensetzung einen pH-Wert von 6,0 bis 8,5 aufweist, vorzugsweise einen pH-Wert von 6,5 bis 8,5, vorzugsweise einen pH-Wert von 7,0 bis 8,0, stärker bevorzugt einen pH-Wert von 8,0.

7. Zusammensetzung nach einem der vorhergehenden Ansprüche, wobei der eine oder die mehreren GLP-1/GLP-2-Dualagonisten der allgemeinen Formel B entsprechen:

H[Aib]EG-X5-FT-SELATILD-[Ψ]-QAARDFIAWLI-X28-HKITD (B),

wobei X5 T oder S ist; X28 Q, E, A, H, Y, L, K, R oder S ist und
wobei [Ψ] einen L- oder D-Lysinrest andeutet, in dem ein Albuminbindungsmolekülteil an den GLP-1/GLP-2-Dualagonisten konjugiert ist, und
wobei der Albuminbindungsmolekülteil [K([17-carboxy-heptadecanoyl]-isoGlu)] ist.

8. Zusammensetzung nach einem der vorhergehenden Ansprüche, wobei der eine oder die mehreren GLP-1/GLP-2-Dualagonisten die folgende Sequenz umfassen:
H[Aib]EGSFTSELATILD[Ψ]QAARDFIAWLIQHKITD (SEQ ID NO: 1).

9. Zusammensetzung nach einem der vorhergehenden Ansprüche, wobei der eine oder die mehreren GLP-1/GLP-2-Dualagonisten sind:

Hy-H[Aib]EGSFTSELATILD[K([17-carboxy-heptadecanoyl]-isoGlu)]QAARDFIAWLIQHKITD-OH (CPD1OH);

oder

Hy-H[Aib]EGSFTSELATILD[K([17-carboxy-heptadecanoyl]-isoGlu)]QAARDFIAWLIQHKITD-$NH_2$ (CPD1$NH_2$),

oder ein pharmazeutisch annehmbares Salz von CPD1OH oder CPD1$NH_2$, vorzugsweise ein Chloridzsalz von CPD1OH oder CPD1$NH_2$.

10. Zusammensetzung nach einem der vorhergehenden Ansprüche, wobei der GLP-1/GLP-2-Dualagonist in einer Konzentration von mindestens 1 mg/ml vorliegt, vorzugsweise in einer Konzentration von 1 mg/ml bis 33 mg/ml, wie etwa in einer Konzentration von 2 mg/ml bis 33 mg/ml, wie etwa in einer Konzentration von 1 mg/ml bis 25 mg/ml, wie etwa in einer Konzentration von 6 mg/ml bis 25 mg/ml.

11. Zusammensetzung nach Anspruch 10, wobei der GLP-1/GLP-2-Dualagonist in einer Konzentration von 2 mg/ml, 15

mg/ml oder 25 mg/ml vorliegt.

**12.** Zusammensetzung nach einem der vorhergehenden Ansprüche, wobei die Zusammensetzung ferner ein oder mehrere Tonizitätsmittel umfassen.

**13.** Zusammensetzung nach Anspruch 12, wobei das eine oder die mehreren Tonizitätsmittel Mannitol umfassen oder sind, vorzugsweise D-Mannitol.

**14.** Zusammensetzung nach Anspruch 13, wobei das Mannitol in einer Konzentration von 130 mM bis 330 mM vorliegt, vorzugsweise von 150 mM bis 300 mM, vorzugsweise von 190 mM bis 240 mM, vorzugsweise 230 mM.

**15.** Zusammensetzung nach Anspruch 12, wobei das eine oder die mehreren Tonizitätsmittel NaCl umfassen oder sind, vorzugsweise wobei das NaCl in einer Konzentration von 50 mM bis 450 mM vorliegt, vorzugsweise von 65 mM bis 165 mM, vorzugsweise 125 mM.

**Revendications**

**1.** Composition comprenant :

(a) un ou plusieurs agonistes doubles de GLP-1/GLP-2 comprenant la formule générale A :

H[Aib]EG-X5-F-X7-SELATILD-[Ψ]-QAARDFIAWLI-X28-HKITD (A),

dans laquelle X5 est T ou S ; X7 est T ou S ; X28 est Q, E, A, H, Y, L, K, R ou S et au moins un parmi X5 et X7 est T,
dans laquelle [Ψ] indique un résidu de D ou L lysine dans laquelle un groupement de liaison à l'albumine est conjugué à l'agoniste double de GLP-1/GLP-2, et
dans laquelle ledit groupement de liaison à l'albumine est [K([17-carboxy-heptadécanoyl]-isoGlu)] ;

(b) un ou plusieurs conservateurs, dans laquelle le ou les conservateurs comprennent ou sont le m-crésol et/ou le phénol ; et
(c) un tampon tris.

**2.** Composition selon la revendication 1, dans laquelle la composition est une composition pharmaceutique parentérale isotonique.

**3.** Composition selon l'une quelconque des revendications précédentes, dans laquelle le ou les conservateurs comprennent ou sont le m-crésol, de préférence dans laquelle le m-crésol est présent à une concentration de 1,15 mg/mL à 5,15 mg/mL, plus préférablement dans laquelle le m-crésol est présent à une concentration de 3,15 mg/mL.

**4.** Composition selon l'une quelconque des revendications précédentes, dans laquelle le ou les conservateurs comprennent ou sont le phénol, de préférence dans laquelle le phénol est présent à une concentration de 2,5 mg/mL à 8,5 mg/mL, plus préférablement dans laquelle le phénol est présent à une concentration de 5,5 mg/mL.

**5.** Composition selon l'une quelconque des revendications précédentes, dans laquelle le tampon tris est présent à une concentration de 5 mM à 50 mM, de préférence dans laquelle le tampon tris est présent à une concentration de 20 mM.

**6.** Composition selon l'une quelconque des revendications précédentes, dans laquelle la composition a un pH allant de pH 6,0 à pH 8,5, de préférence un pH allant de pH 6,5 à pH 8,5, de préférence un pH allant de pH 7,0 à pH 8,0, plus préférablement un pH de pH 8,0.

**7.** Composition selon l'une quelconque des revendications précédentes, dans laquelle l'agoniste ou les agonistes doubles de GLP-1/GLP-2 sont de la formule générale B :

H[Aib]EG-X5-FT-SELATILD-[Ψ]-QAARDFIAWLI-X28-HKITD (B),

dans laquelle X5 est T ou S ; X28 est Q, E, A, H, Y, L, K, R ou S et
dans laquelle [Ψ] indique un résidu de D ou L lysine dans laquelle un groupement de liaison à l'albumine est conjugué à l'agoniste double de GLP-1/GLP-2 et
dans laquelle ledit groupement de liaison à l'albumine est [K([17-carboxy-heptadécanoyl]-isoGlu)].

**8.** Composition selon l'une quelconque des revendications précédentes, dans laquelle l'agoniste ou les agonistes doubles de GLP-1/GLP-2 comprennent la séquence :
H[Aib]EGSFTSELATILD[Ψ]QAARDFIAWLIQHKITD (SEQ ID NO: 1).

**9.** Composition selon l'une quelconque des revendications précédentes, dans laquelle l'agoniste ou les agonistes doubles de GLP-1/GLP-2 sont :

Hy-H[Aib]EGSFTSELATILD[K([17-carboxy-heptadécanoyl]-isoGlu)]QAARDFIAWLIQHKITD-OH (CPD10H) ;

ou

Hy-H[Aib]EGSFTSELATILD[K([17-carboxy-heptadécanoyl]-isoGlu)]QAARDFIAWLIQHKITD-$NH_2$ (CPD1$NH_2$),

ou un sel pharmaceutiquement acceptable de CPD10H ou CPD1$NH_2$, de préférence un sel chlorure de CPD10H ou CPD1$NH_2$.

**10.** Composition selon l'une quelconque des revendications précédentes, dans laquelle l'agoniste double de GLP-1/GLP-2 est présent à une concentration d'au moins 1 mg/mL, de préférence une concentration de 1 mg/mL à 33 mg/mL, telle qu'une concentration de 2 mg/mL à 33 mg/mL, telle qu'une concentration de 1 mg/mL à 25 mg/mL, telle qu'une concentration de 6 mg/mL à 25 mg/mL.

**11.** Composition selon la revendication 10, dans laquelle l'agoniste double de GLP-1/GLP-2 est présent à une concentration de 2 mg/mL, 15 mg/mL ou 25 mg/mL.

**12.** Composition selon l'une quelconque des revendications précédentes, dans laquelle la composition comprend en outre un ou plusieurs agents de tonicité.

**13.** Composition selon la revendication 12, dans laquelle l'agent ou les agents de tonicité comprennent ou sont le mannitol, de préférence le D-mannitol.

**14.** Composition selon la revendication 13, dans laquelle le mannitol est présent à une concentration de 130 mM à 330 mM, de préférence de 150 mM à 300 mM, de préférence de 190 mM à 240 mM, de préférence de 230 mM.

**15.** Composition selon la revendication 12, dans laquelle l'agent ou les agents de tonicité comprennent ou sont NaCl, de préférence dans laquelle le NaCl est présent à une concentration de 50 mM à 450 mM, de préférence de 65 mM à 165 mM, de préférence de 125 mM.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018104561 A
- WO 9811125 A


### Non-patent literature cited in the description


- **BERGE et al.** *J. Pharm. Sci.*, 1977, vol. 66, 1-19
- Principles and practice of solid-phase peptide synthesis. **FIELDS, G.B et al.** Synthetic Peptides. 2002
- **VINCENT LEE**. Peptide and Protein Drug Delivery. Marcel Dekker, Inc., 1991, 247-301
- **JONES, A.** Adv. Drug Delivery Rev.. 1993, vol. 10, 29-90